# EUROPEAN PATENT APPLICATION

(11) **EP 2 859 840 A1**
(43) Date of publication of application: **15.04.2015**
(21) Application number: 14188286.0
(22) Date of filing: 09.10.2014
(51) Int. Cl.: A61B 5/00, A44C 5/00, A44C 5/18, A44C 5/20

(54) **Band and biological information measurement device**

(30) Priority: 11.10.2013 JP 2013213479
(71) Applicant: Seiko Epson Corporation, Shinjuku-ku Tokyo (JP)
(72) Inventor: Shibuya, Junya, Nagano, 392-8502 (JP); Hidai, Yoshihiro, Nagano, 392-8502 (JP); Tanaka, Shigemitsu, Nagano, 392-8502 (JP)
(74) Representative: Hoffmann Eitle

(57) **Abstract**

A band for mounting a device main body includes first and second band portions which are connected to device main body, wherein the ratio between the widths of the first band portion and the second band portion in a second direction intersecting a first direction in which the first band portion and the second band portion extend from the device main body and the width of the device main body in the second direction is greater than or equal to 0.5 and less than or equal to 1.4.

## Description

### BACKGROUND

### 1. Technical Field

The present invention relates to a band for mounting a timepiece or a measurement device main body of an electronic measurement device which measures biological information on the wrist or the like of a wearer, and a biological information measurement device which is provided with the band.

### 2. Related Art

In the past, an electronic device such as a measurement device which is mounted on the wrist or the like of a wearer and measures biological information such as the pulse or a wristwatch having a function of measuring the biological information has been known. In such an electronic device, it is necessary to bring a device main body which performs measurement into close contact with an appropriate site such as a wrist of the wearer. A band which is mounted on a main body of such a measurement device or electronic device and provided with a stretchable belt portion is disclosed in JP-A-2012-90975.

The band described in JP-A-2012-90975 is provided with first and second band members which are mounted on a device main body, and a connecting member. Each band member has a stretchable portion which expands and contracts along a band extension direction, and the stretchable portion has flexibility and has a first slit having a pair of slits following a width direction and a slit following the band extension direction, and a pair of second slits communicating with the outside of the stretchable portion along the width direction. The connecting member is provided with a fixed member, a sliding member slidable along the band extension direction of the first band member, and a biasing member which biases the sliding member in the opposite direction to the band extension direction, and the sliding member is provided with a connection portion (a projecting bar) which is connected to the second band member.

When mounting such a biological information measurement device on the wrist of a test object, a tensile force acts on the band by an operation of a wearer, and a device main body is mounted on the wrist in a state where the stretchable portion is extended by the tensile force. Then, if the wearer removes the band from the hand, a restoring force acts on the stretchable portion. In this way, the band is tightened up, and thus the device main body can be brought into close contact with the wrist regardless of the degree of tightening of the device main body to the wrist by the wearer.

In addition, in JP-A-2012-90975, as the stretchable portion, a structure is shown in which an elastic material such as urethane or silicone is provided in a meandering fashion in a direction intersecting a direction in which a band member extends.

However, the band described in JP-A-2012-90975 described above is thin compared to the width of the device main body in a case of being viewed in plan from a vertical direction with respect to a display surface of a display section provided at the portable biological information measurement device and requires a great tensile force and a restoring force of the stretchable portion in order to stably mount the device main body. Therefore, a feeling of pressure is given to the wrist or the like of a wearer by the restoring force of the stretchable portion and a feeling of discomfort is given to prolonged mounting. Further, the device main body section is greatly visible, and therefore, there is also a problem in that a visual feeling of pressure is given to the wearer. Therefore, there is also a concern that a feeling of pressure on a wrist or the like and a visual sensation may adversely affect the biological information of a measurement object.

### SUMMARY

An advantage of some aspects of the invention is to solve at least a part of the problems described above, and the invention can be implemented as the following forms or application examples.

### Application Example 1

This application example of the invention is directed to a band for mounting a device main body including: first and second band portions which are connected to a device main body, wherein the ratio between the widths of the first band portion and the second band portion in a second direction intersecting a first direction in which the first band portion and the second band portion extend from the device main body and the width of the device main body in the second direction is greater than or equal to 0.5 and less than or equal to 1.4.

According to such a band, the ratio between the widths of the first and second band portions connected to the device main body and the width of the device main body is greater than or equal to 0.5 and less than or equal to 1.4. Therefore, a feeling of pressure occurring due to the widths of the device main body and the band becoming too thin is suppressed and a feeling of fatigue occurring due to the widths of the device main body and the band becoming too thick is suppressed, whereby it is possible to stably mount the device main body. Further, it is possible to make the device main body be viewed as small, and thus it is possible to alleviate a visual feeling of pressure. Therefore, the influence on the biological information of a test object on which the device main body is mounted is alleviated, and thus it is possible to obtain biological information with high precision by the device main body mounted by the band.

### Application Example 2

In the band according to the application example described above, it is preferable that the band further includes: a connection portion which is provided at an end portion of the first band portion on the opposite side to the device main body and connects the first band portion and the second band portion; and a hook portion which is provided at an end portion of the second band portion on the opposite side to the device main body and locks the second band portion to the first band portion.

According to such a band, the second band portion is connected to the first band portion by the connection portion provided at the first band portion, and an end portion of the connected second band portion is locked to the first band portion by the hook portion. Therefore, the first band portion and the second band portion are connected in the form of a ring, and play of the end portion of the connected second band portion can be suppressed. Therefore, when mounting the device main body mounted on the band, release of the connection between the first band portion and the second band portion due to being caught in clothes or the like of a test object with the device main body mounted thereon, and detachment of the device main body can be suppressed.

### Application Example 3

In the band according to the application example described above, it is preferable that belt portions having stretchability is provided between an end portion of the first band portion, which is connected to the device main body, and the connection portion and between an end portion of the second band portion, which is connected to the device main body, and the hook portion.

According to such a band, the belt portions having stretchability are provided. Therefore, by extending and mounting the band when mounting the device main body, it is possible to improve mountability by a force when the belt portions contract and are restored. Therefore, it is possible to increase close contact with a person on which the device main body is mounted, thereby suppressing a shift of the mounted device main body.

### Application Example 4

In the band according to the application example described above, it is preferable that in the belt portions, hole portions in which the connection portion or the hook portion is inserted are provided in parallel in the second direction and to form rows in the first direction.

According to such a band, the hole portions are provided in parallel and to form rows in the belt portions. Therefore, a position where the connection portion is inserted can be adjusted according to the position of the hole portion. Therefore, it is possible to connect the first band portion and the second band portion at an optimum position in accordance with a test object on which the device main body is mounted. Further, the end portion of the second band portion connected to the first band portion can be locked to the first band portion by inserting the hook portion into the hole portion. Therefore, the end portion of the second band portion can be locked without any play.

### Application Example 5

In the band according to the application example described above, it is preferable that in each of the hole portions, first and second holes having an elliptical shape having a short axis in the first direction and a long axis in the second direction are coaxially provided and the short axis and the long axis of the second hole are larger than the short axis and the long axis of the first hole.

According to such a band, the hole portion has an elliptical shape, whereby, when connecting the first band portion and the second band portion, it is easy to insert a projecting bar, and when the projecting bar is pressed against the hole portion according to the expansion and contraction of the belt portion, deformation of the hole portion can be suppressed. Further, the first hole and the second hole, the short axis and the long axis of which are larger than those of the first hole, are coaxially provided, whereby it is possible to suppress the hook portion locking the second band portion from protruding from the first band portion. Therefore, it is possible to suppress deformation of the hole portion due to being mounted and suppress a feeling of discomfort which is given to a wearer due to protrusion of the hook portion. Therefore, it is possible to realize a band which is easy to be constantly mounted for a long period of time.

### Application Example 6

In the band according to the application example described above, it is preferable that in the rows of the hole portions of the first band portion, a distance between a row closest to the device main body side and a row adjacent thereto is narrower than a distance between a row closest to the connection portion side and a row adjacent thereto.

According to such a band, intervals of the hole portions in a row direction are provided so as to be narrow on the connection portion side, compared to an interval of the device main body side. Therefore, when connecting the first band portion and the second band portion and locking the second band portion to the first band portion by the hook portion, it is possible to finely perform the adjustment of a locking position of the second band portion by the hook portion.

### Application Example 7

In the band according to the application example, it is preferable that in the rows of the hole portions of the second band portion, a distance between a row closest to the device main body side and a row adjacent thereto is narrower than a distance between a row closest to the hook portion side and a row adjacent thereto.

According to such a band, intervals of the hole portions in the row direction are provided so as to be narrow on the hook portion side, compared to an interval of the device main body side. Therefore, when connecting the first band portion and the second band portion by the connection portion, it is possible to finely perform the adjustment of a connection position between the first band portion and the second band portion. Therefore, it is possible to easily adjust the strength of mounting the device main body on a test object.

### Application Example 8

In the band according to the application example described above, it is preferable that the first band portion has a thick portion, the connection portion is provided at the thick portion and includes a band insertion hole in which the second band portion is inserted, and a projecting bar which is pivotally supported on one side of the band insertion hole, which extends in the second direction, and is inserted into the hole portion provided in the second band portion, and the band insertion hole is provided such that a side extending in the second direction, which faces the one side on which the projecting bar is pivotally supported, is long.

According to such a band, the band insertion hole in which the second band portion is inserted is provided such that the side extending in the second direction, which faces the one side on which the projecting bar is pivotally supported, is long. Therefore, it is possible to insert the second band portion into the band insertion hole and easily extend the belt portion configuring the second band portion. Therefore, it is possible to realize a band in which it is possible to stably mount the device main body on a test object by a restoring force of the belt portion which contracts.

### Application Example 9

In the band according to the application example described above, it is preferable that the hook portion is provided with a base portion and a pin portion extending from the base portion and the pin portion has a first shaft portion extending from the base portion, and a second shaft portion extending from the first shaft portion.

According to such a band, in the hook portion, the first shaft portion extends from the base portion and the second shaft portion extends from the first shaft portion. Therefore, the hook portion is mounted on the second band portion by inserting the first shaft portion into the hole portion provided in the second band portion, and the first shaft portion extending from the second shaft portion can be inserted into the hole portion provided in the first band portion. Therefore, it is possible to lock the second band portion to the first band portion.

### Application Example 10

In the band according to the application example, it is preferable that in the pin portion, a first head portion having a larger diameter than the first shaft portion is provided at the first shaft portion and a second head portion having a larger diameter than the second shaft portion is provided at the second shaft portion.

According to such a band, the first shaft portion is inserted into the hole portion provided in the second band portion and the first head portion is caught in the hole portion, whereby it is possible to suppress the hook portion from being detached from the second band portion. Further, the second shaft portion is inserted into the hole portion provided in the first band portion and the second head portion is caught in the hole portion, whereby it is possible to lock the second band portion to the first band portion. Therefore, it is possible to suppress detachment of the hook portion and the locked second band portion.

### Application Example 11

In the band according to the application example described above, it is preferable that a length of the second shaft portion in a third direction in which the pin portion extends from the base portion is approximately the same as a depth of the first hole and a length of the second head portion in the third direction is approximately the same as a depth of the second hole.

According to such a band, since the length of the second shaft portion is approximately the same as the depth of the first hole and the length of the second head portion is approximately the same as the depth of the second hole, it is possible to suppress the second shaft portion and the second head portion inserted into the hole portion provided in the first band portion from protruding from the first band portion. Therefore, it is possible to suppress a feeling of discomfort which is given to a wearer by protrusion of the hook portion. Therefore, it is possible to provide a band which is easy to be constantly mounted for a long period of time.

### Application Example 12

In the band according to the application example described above, it is preferable that the second shaft portion and the second head portion have an elliptical shape having a short axis in the first direction and a long axis in the second direction and an area of the second head portion when the second head portion and the first hole are viewed in plan from the third direction is larger than an area of the first hole.

According to such a band, in the pin portion inserted into the hole portion provided in the first band portion, the area of the second head portion is larger than the area of the first hole, and therefore, the second head portion is caught in the first hole, and thus it is possible to suppress the pin portion from easily coming out of the hole portion. Therefore, it is possible to lock the second band portion to the first band portion and suppress the second band portion from being detached from the first band portion.

### Application Example 13

In the band according to the application example described above, it is preferable that the second shaft portion and the second head portion have an elliptical shape having a short axis in the first direction and a long axis in the second direction and the long axis and the short axis of the second head portion are larger than the long axis and the short axis of the first hole.

According to such a band, in the pin portion inserted into the hole portion provided in the first band portion, the short axis and the long axis of the second head portion are larger than the short axis and the long axis of the first hole, and therefore, the second head portion is caught in the first hole, and thus it is possible to suppress the pin portion from easily coming out of the hole portion. Therefore, it is possible to lock the second band portion to the first band portion and suppress the second band portion from being detached from the first band portion.

### Application Example 14

In the band according to the application example, it is preferable that each of the first head portion and the second head portion has a curved surface in the opposite direction to a direction in which the base portion is provided.

According to such a band, each of the first head portion and the second head portion has a curved surface in the opposite direction to a direction in which the base portion is provided, whereby it is possible to suppress contact resistance when inserting the pin portion having the first shaft portion and the second shaft portion into the hole portion. Therefore, the force when locking the second band portion to the first band portion requires a larger force when extracting the pin portion, that is, when detaching the first band portion from the second band portion than when inserting the pin portion into the first band portion. Therefore, it is possible to lock the second band portion to the first band portion and suppress the second band portion from being detached from the first band portion.

### Application Example 15

In the band according to the application example described above, it is preferable that in the belt portions, a thickness in the third direction is approximately the same as lengths of the second shaft portion and the second head portion.

According to such a band, the thickness of the belt portion is approximately the same as the value obtained by adding the lengths of the second shaft portion and the second head portion, whereby it is possible to lock the second band portion to the first band portion without protrusion of the second head portion from the belt portion.

### Application Example 16

In the band according to the application example described above, it is preferable that in the first band portion and the second band portion, the belt portions are formed of polyurethane having stretchability.

According to such a band, it is possible to increase a close contact force of the device main body with a wearer by a restoring force occurring by mounting the device main body with the band mounted thereon by extending the belt portion, and then releasing a tensile force extended. Further, by using polyurethane for the belt portion, compared to a band using a belt portion made of metal, a reduction in weight is attained, and thus it is possible to alleviate a feeling of pressure to a wearer of the device main body. Therefore, it is possible to suppress a feeling of discomfort which is given to the wearer. Therefore, it is possible to provide a band which is easy to be constantly mounted.

### Application Example 17

This application example of the invention is directed to a biological information measurement device including: the band described above; and a device main body on which the band is mounted.

According to such a biological information measurement device, the width of the band mounted on the device main body has approximately the same width as the device main body. Therefore, the device main body and the band are in contact with a test object to have approximately the same contact area, and thus a feeling of pressure to a wrist or the like due to prolonged mounting is suppressed, and thus it is possible to stably mount the device main body. Further, the device main body can be viewed as small, and thus a feeling of pressure can be alleviated also in terms of a visual sensation. Therefore, it is possible to realize a biological information measurement device in which the influence on the biological information of a test object on which the device main body is mounted is alleviated, and thus it is possible to obtain biological information with high precision by the device main body mounted by the band.

### BRIEF DESCRIPTION OF THE DRAWINGS

The invention will be described with reference to the accompanying drawings, wherein like numbers reference like elements.
Fig. 1 is a top view schematically showing the external appearance of a biological information measurement device with a band according to an embodiment mounted thereon.
Figs. 2A and 2B are perspective views schematically showing the external appearance of the biological information measurement device with the band according to the embodiment mounted thereon.
Fig. 3 is a development view schematically showing the structure of the biological information measurement device with the band according to the embodiment mounted thereon.
Figs. 4A and 4B are a plan view and a cross-sectional view schematically showing a first band portion.
Figs. 5A and 5B are cross-sectional views schematically showing the cross-section of a hole portion of the first band portion on an enlarged scale.
Figs. 6A to 6C are a plan view and cross-sectional views schematically showing a second band portion.
Figs. 7A to 7C are cross-sectional views schematically showing the cross-section of a hole portion of the second band portion on an enlarged scale.
Figs. 8A and 8B are enlarged views showing a hook portion provided at the band according to the embodiment on an enlarged scale.

### DESCRIPTION OF EXEMPLARY EMBODIMENTS

Hereinafter, an embodiment of the invention will be described using the drawings. In addition, in each drawing shown below, in order to show each constituent element in size to the extent that can be recognized on the drawing, there is a case where the dimension or the ratio of each constituent element is described differently from that of the actual constituent element.

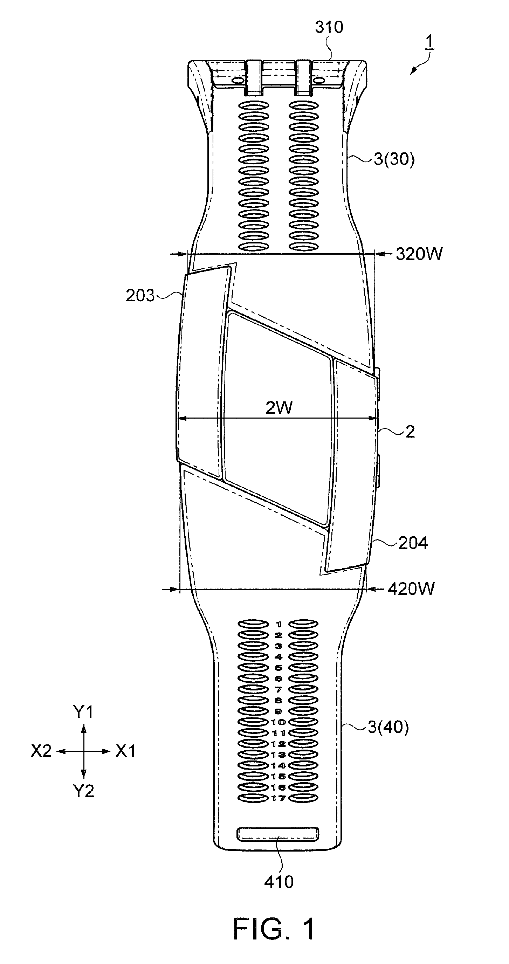

A band according to this embodiment and a portable biological information measurement device will be described using Figs. 1 to 8B.

Fig. 1 is a top view schematically showing the external appearance of a biological information measurement device with a band according to an embodiment mounted thereon.

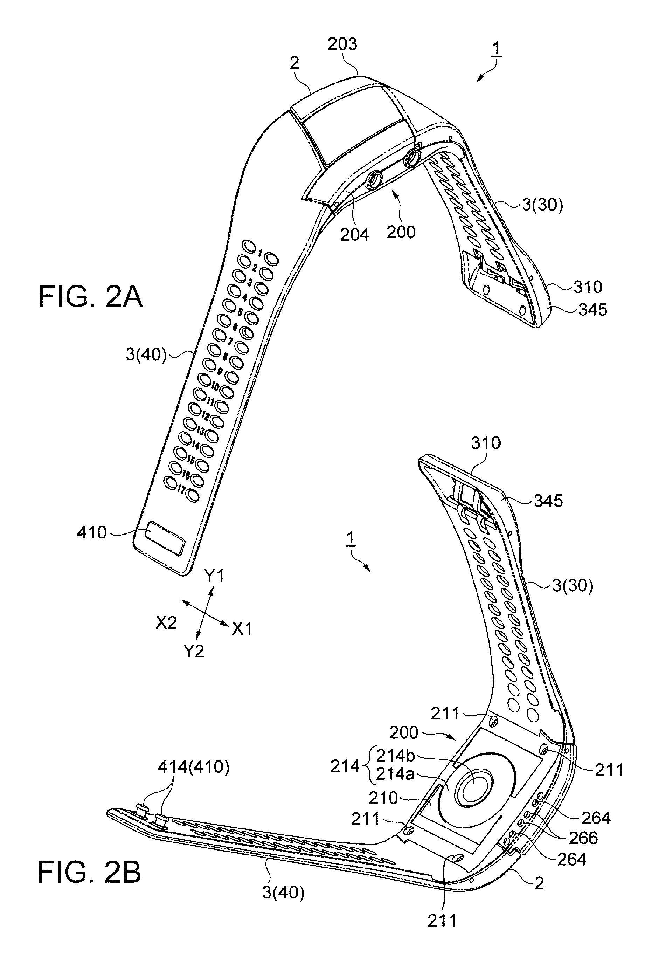

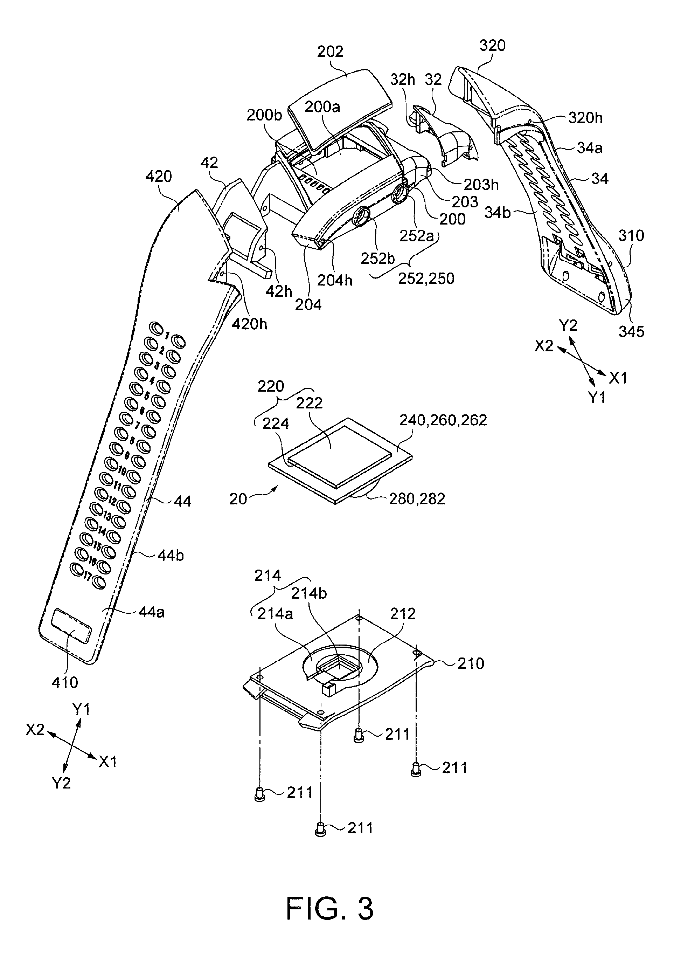

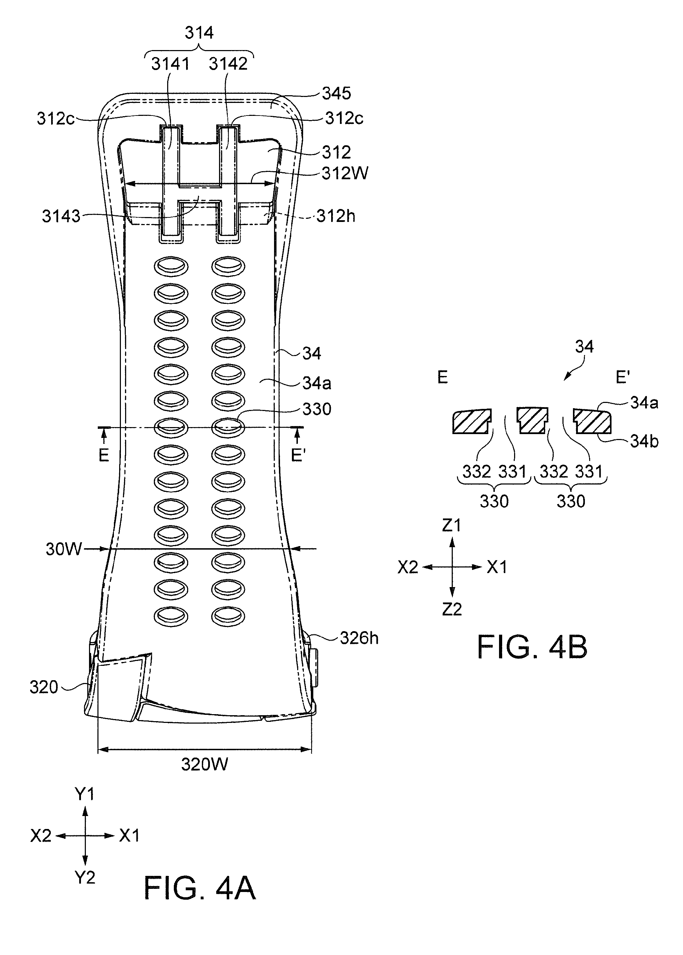

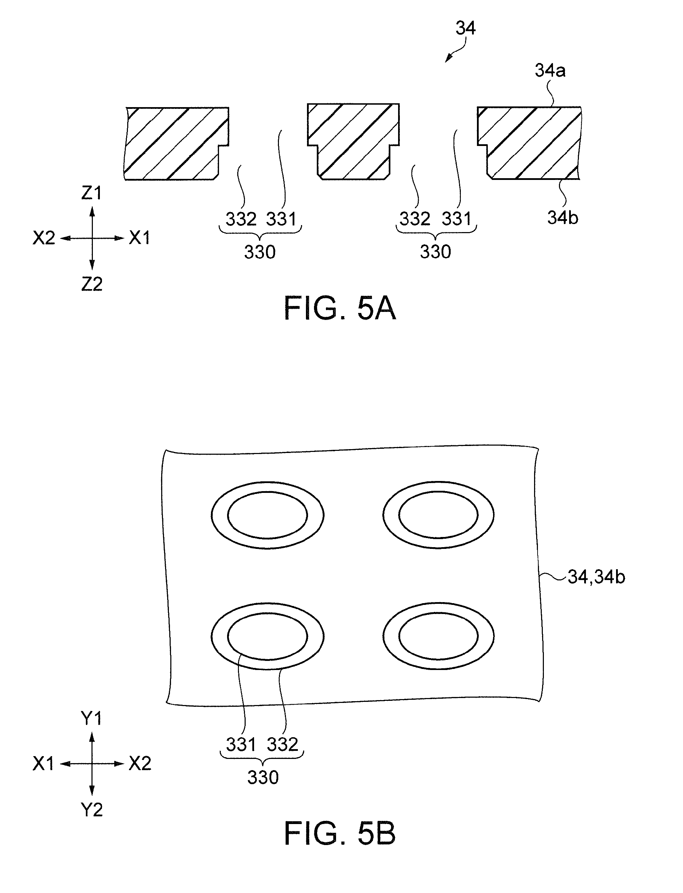

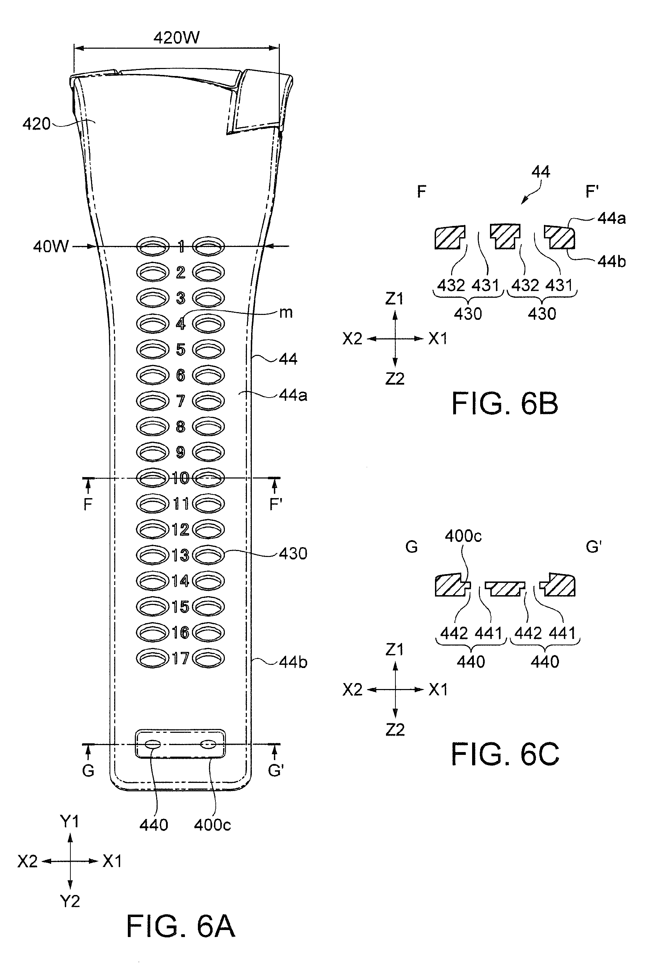

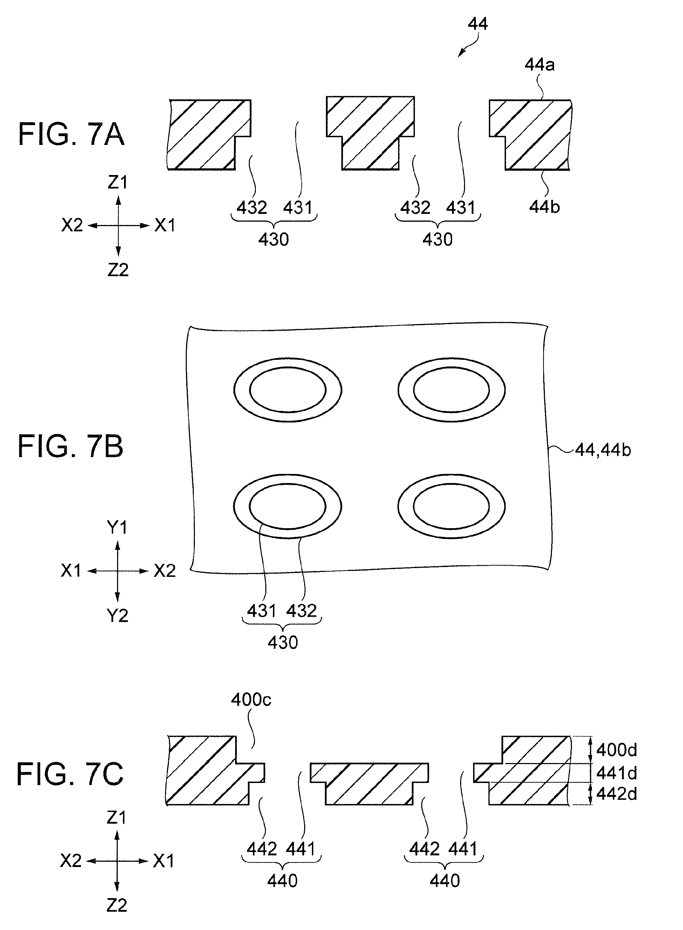

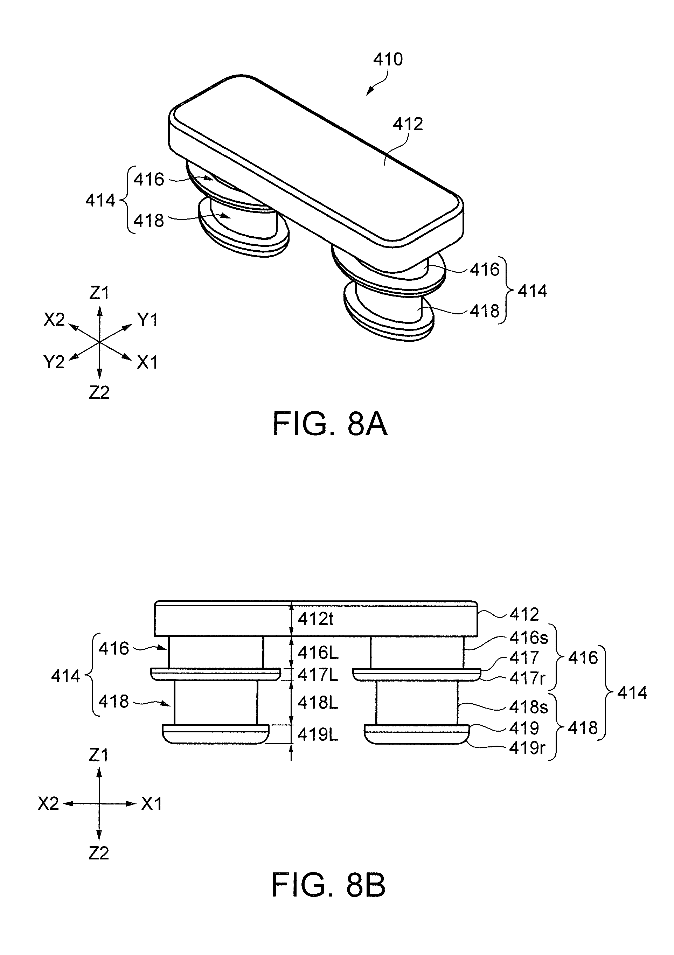

Figs. 2A and 2B are perspective views schematically showing the external appearance of the biological information measurement device with the band mounted thereon. Fig. 3 is a development view schematically showing the structure of the biological information measurement device with the band mounted thereon. Figs. 4A and 4B are a plan view and a cross-sectional view schematically showing a first band portion. Figs. 5A and 5B are cross-sectional views schematically showing the cross-section of a hole portion of the first band portion on an enlarged scale. Figs. 6A to 6C are a plan view and cross-sectional views schematically showing a second band portion. Figs. 7A to 7C are cross-sectional views schematically showing the cross-section of a hole portion of the second band portion on an enlarged scale. Figs. 8A and 8B are enlarged views showing a hook portion provided at the band on an enlarged scale.

### Schematic Configuration of Biological Information Measurement Device 1

A biological information measurement device 1 (hereinafter referred to simply as a "measurement device 1") according to this embodiment is an electronic device which is mounted on the wrist or the like of a test object (for example, a human body), biological information of which is measured, and measures biological information such as the pulse. The measurement device 1 is formed to have an external appearance similar to a wristwatch, as shown in Figs. 1, 2A, and 2B. The measurement device 1 is provided with a device main body 2 which comes into close contact with a test object, thereby measuring biological information, and a band section 3 which is mounted on the device main body 2.

### Configuration of Device Main Body 2

The device main body 2 of the measurement device 1 shown in Figs. 2A, 2B, and 3 is provided with a module 20, and a case section 200 in which the module 20 is accommodated. The module 20 is provided with a display section 220, a processing section 240, a power supply section 260, and a sensor section 280.

In the case section 200, a concave portion 200b in which the module 20 is accommodated is formed on the lid section 210 side thereof. The module 20 is accommodated in the concave portion 200b and the lid section 210 is disposed so as to cover the concave portion 200b and is fixed by setscrews 211. Materials of the case section 200 and the lid section 210 are not particularly limited. In this embodiment, as an example thereof, nylon-based synthetic resin (plastic resin) in which the occurrence of cracks are hard is used.

The display section 220 configuring the module 20 is provided at the case section 200.

In the display section 220, a display panel 222 is provided and biological information measurement data such as the number of pulses, time information such as the current time, or the like is displayed according to each display mode. Further, a backlight 224 is provided in the display section 220 and can illuminate the display panel 222.

As long as the display panel 222 can display biological information (mainly, a numeral or a graph configured by a dot matrix) such as the pulse, a display method is not limited. In the measurement device 1, as an example thereof, a liquid crystal display device is used.

Further, as for the backlight 224, as long as it is possible to perform illumination to the extent that biological information which is displayed on the display panel 222 is visible, a luminescence method or a luminescent color is not limited. In the measurement device 1, the illumination of the display panel 222 is performed by using an electro-luminescence (EL) panel which emits green light.

In the case section 200, a display window 200a is provided on the opposite side to the side on which the lid section 210 is provided. The display window 200a is configured such that biological information or the like which is displayed on the display section 220 is visible. Further, a display section cover 202 formed of transparent resin, transparent glass, or the like is fitted into the display window 200a, and the display section 220 is protected by the display section cover 202.

The processing section 240 configuring the module 20 is provided in the case section 200.

The processing section 240 is a substrate configured by a semiconductor device such as a microcomputer, or a storage device. The display section 220, an operating section 250, the power supply section 260, the sensor section 280 are connected to the processing section 240. The processing section 240 processes the driving of the sensor section 280 or a signal based on the pulse detected in the sensor section 280, thereby performing the display processing of biological information or the like in the display section 220. Further, the processing section 240 performs the storing of biological information and performs communication with an information processing device (not shown) provided outside the measurement device 1, thereby being able to output the stored data.

The operating section 250 providing a command to the processing section 240 is provided in the case section 200.

In the operating section 250, an operation button 252 capable of being pressed is provided. By pressing the operation button 252, it is possible to perform switching of a pulse measurement mode of displaying, for example, pulse measurement data, a clock mode of displaying the current time or the like, a remaining battery level display mode, a lighting mode of the backlight 224 of the display section 220, or the like.

Further, the operation button 252 can be provided in a plurality. By providing the plurality of operation buttons 252 and pressing the operation buttons 252 at substantially the same time, it is possible to perform switching to a setting mode of performing the setting of the measurement device 1. In the measurement device 1 according to this embodiment, as an example thereof, two operation buttons 252a and 252b are provided in the measurement device 1. An operation of the operating section 250 by a test object can be performed by pressing the operation button 252a by an index finger and adding a force associated with the pressing to the case section 200 through a thumb.

Further, an operation can be performed by pressing the operation button 252b by a ring finger and adding a force associated with the pressing to the case section 200 through a thumb. Therefore, it is preferable that an interval at which the operation buttons 252a and 252b are provided is an interval in which fingers (for example, a thumb and a ring finger) performing the operation do not overlap, in a range that the fingers performing the operation reach.

A charging terminal 264 which is used in the charging of a battery 262 installed in the power supply section 260 which serves as the power supply of the measurement device 1 is provided in the case section 200. In addition, a communication terminal 266 which is used in data communication of biological information (data) measured and stored in the measurement device 1, measurement setting data, and the like is provided in the case section 200.

In addition, the battery 262 is not limited to a rechargeable secondary battery and a primary battery such as a lithium battery can be used.

A sensor unit 282 is provided in the sensor section 280. The sensor unit 282 is a light sensor and is provided with a sensor case, and a sensor substrate with a light emitting element and a light receiving element mounted thereon. The sensor unit 282 irradiates light toward the wrist of a test object from the light emitting element such as a light emitting diode (LED) and receives the light reflected by a blood vessel of the wrist by the light receiving element such as a photodiode.

A sensor bank portion 212 in which the sensor section 280 is accommodated is provided at the lid section 210. The sensor section 280 is provided on the sensor bank portion 212 having a disk shape raised from the lid section 210 in the opposite direction to a display direction of the display section 220. The sensor bank portion 212 is provided with a sensor convex portion 214.

The sensor convex portion 214 is provided so as to be pressed against (be brought into contact with) the wrist or the like of a test object with the measurement device 1 mounted thereon. The sensor convex portion 214 is provided with a base portion 214a extending from the lid section 210, and a tip portion 214b which is pressed against a test object.

From this, it is preferable that the base portion 214a of the sensor convex portion 214 is made of a material having a light shielding property. Further, it is preferable that the tip portion 214b is made of a material capable of transmitting light which is emitted from the light emitting element. Further, it is preferable that the tip portion 214b has a shape which suppresses irregular reflection of the light and in which a test object does not feel pain at the time of mounting.

Therefore, the base portion 214a is provided by using the same synthetic resin as the lid section 210 and applying light-shielding coloring thereto. Further, the tip portion 214b is provided to have an arc shape by using transparent glass or transparent acrylic resin.

### Configuration of Band Section 3

Returning to Figs. 1, 2A, and 2B, the configuration of the band section 3 will be described. The band section 3 is provided in order to mount the device main body 2 on a test object.

The bands 3 are provided at both ends of the device main body 2, as shown in Figs. 1, 2A, and 2B. A first band portion 30 configuring the band section 3 is mounted on a lug 203 (a lug on the twelve o'clock side in a timepiece as a first direction) of the device main body 2 by a mounting member 32. Further, a second band portion 40 configuring the band section 3 is mounted on a lug 204 (a lug on the six o'clock side in a timepiece as the first direction) of the device main body 2 by a mounting member 42.

A connection portion 310 which connects the first band portion 30 and the second band portion 40 is provided at the first band portion 30. The connection portion 310 is provided at an end portion of the first band portion 30 on the opposite side to the device main body 2 side. Further, a hook portion 410 which locks the second band portion 40 to the first band portion 30 is provided at the second band portion 40. The hook portion 410 is provided at an end portion of the first band portion 30 on the opposite side to the device main body 2 side.

In the following description, in the first band portion 30, the device main body 2 side is described as being referred to as "one end side" and the side on which the connection portion 310 is provided on the opposite side thereto is described as being referred to as the "other end side". Similarly, in the second band portion 40, the device main body 2 side is described as being referred to as "one end side" and the side on which the hook portion 410 is provided on the opposite side thereto is described as being referred to as the "other end side".

### Configuration of First Band Portion

The first band portion 30 shown in Figs. 3 and 4A has a belt portion 34, a cover portion 320 as a first connection portion (an end portion) provided on one end side (the device main body 2 side, in a Y2 direction shown in Figs. 3 and 4A) of the belt portion 34, and the connection portion 310 on the other end side (the opposite side to the device main body 2, in a Y1 direction shown in Figs. 3 and 4A).

The belt portion 34 has front and back surfaces. In the following description, the surface coming into contact with a wrist when having been mounted on a test object is described as being referred to as a back surface 34b of the belt portion 34 , and the surface being the opposite surface thereto and being visible when having been mounted is described as being referred to as a front surface 34a of the belt portion 34.

### Mounting on Device Main Body 2

The first band portion 30 is mounted on the device main body 2 such that the cover portion 320 covers the lug 203, with the mounting member 32 sandwiched between the lug 203 and the cover portion 320.

The cover portion 320 and the lug 203 are pivotally supported by inserting a "spring rod" (not shown) into a hole 32h provided in the mounting member 32 and a "rod hole 203h" provided in the lug 203 and locking both ends of the spring rod to locking holes 320h provided in the cover portion 320.

### Belt Portion 34

The belt portion 34 shown in Figs. 4A and 4B has stretchability in order to mount the device main body 2 in close contact with the wrist or the like of a test object. The belt portion 34 is made using a material which includes polyurethane resin or silicone resin, thereby having stretchability and flexibility according to the characteristics of the material.

The belt portion 34 is provided with the thickness (in a Z direction shown in Fig. 4B) of a central portion in a direction of a width 30W increased in a cross-section (a cross-section in line E - E' shown in Fig. 4A) in a direction (a second direction, an X direction shown in Fig. 4A) intersecting a direction (a first direction, the Y1 direction shown in Fig. 4A) in which the first band portion 30 extends from the device main body 2. The belt portion 34 is provided with the thickness of the central portion in the direction of the width 30W increased, whereby strength at the time of expansion and contraction and strength at the time of flexion are secured.

A hole portion 330 (a "child hole" in a timepiece) in which the hook portion 410 provided at the second band portion 40 is locked is provided in the belt portion 34. The hole portions 330 are provided in parallel in the direction of the width 30W (the second direction, the X direction shown in Figs. 4A and 4B) and to be aligned to form rows in a direction (the first direction, the Y direction shown in Fig. 4A) in which the belt portion 34 extends. A line spacing of the hole portions 330 may be disposed so as to be almost constant and may also be disposed such that the line spacing becomes shorter as it goes in a direction of the device main body 2, that is, the Y2 direction from the other end. In other words, the line spacing is disposed such that a distance (a second pitch) between the last row in the Y2 direction and a row adjacent thereto becomes narrow compared to a distance (a first pitch) between the last row of the hole portion 330 on the Y1 direction side and a row adjacent thereto. Or, the line spacing is disposed such that the first pitch is wider than the second pitch. Due to such a configuration, even for a test object in which a site of mounting the measurement device 1 is thin, it is possible to finely adjust tightening, and therefore, it becomes easy to perform setting so as to be able to fix the measurement device 1 with an appropriate strength.

In the hole portion 330, as shown in Figs. 4B and 5A, a hole 331 provided on the front surface 34a side of the belt portion 34 and a hole 332 provided on the back surface 34b side of the belt portion 34 are coaxially provided.

The hole portion 330 has an elliptical shape having a long axis (a major diameter) in the direction of the width 30W (the X direction) of the belt portion 34 and a short axis (a minor diameter) in an orthogonal direction (the Y direction) in which the first band portion 30 extends from the device main body 2, as shown in Fig. 5B. The long axis and the short axis orthogonal to the long axis of the hole portion 330 are provided to be longer (larger) in the hole 332 than in the hole 331.

Further, the depth of each of the holes 331 and 332 is made to be a depth in which a tip of a pin 414 provided at the hook portion 410 (described later) does not protrude from the back surface 34b of the belt portion 34.

In the band section 3 according to this embodiment, the hole portion 330 is provided such that the dimension (the size) of the hole 331 is approximately 4.0 mm in the long axis direction and approximately 2.5 mm in the short axis direction and the dimension (the size) of the hole 332 is approximately 5.0 mm in the long axis direction and approximately 3.5 mm in the short axis direction.

### Connection Portion 310

Returning to Figs. 4A and 4B, the configuration of the connection portion 310 will be described.

The connection portion 310 is provided on the other end side (in the Y1 direction shown in Fig. 4A) of the belt portion 34.

A thick portion 345 in which the thickness of the belt portion 34 is increased compared to a portion in which the hole portions 330 are provided is provided on the other end side of the belt portion 34. The connection portion 310 is provided at the thick portion 345 in which the thickness of the belt portion 34 is increased. A band insertion hole 312 into which the second band portion 40 is inserted and a projecting bar 314 which is inserted into a hole portion 430 (refer to Figs. 6A to 6C) provided in the second band portion 40 are provided at the connection portion 310.

In the connection portion 310, the band insertion hole 312 is provided such that a width 312W in a direction intersecting a direction (the Y1 direction shown in Fig. 4A) in which the first band portion 30 extends from the device main body 2 is wider than a width 40W (refer to Fig. 6A) of the second band portion 40.

More specifically, a width on a side of one side (the Y1 side shown in Fig. 4A) on which a concave portion 312c (described later) is provided is provided to be wider than a width on the side of one side (the Y2 side shown in Fig. 4A) on which a locking hole 312h in which the projecting bar 314 (described later) is locked is provided.

The band insertion hole 312 is provided such that the width on the Y1 side is wider, whereby it is possible to easily insert the second band portion 40 therein. Further, it is possible to increase the close contact of the device main body 2 with a wrist by extending the second band portion 40 by pulling the second band portion 40 to the Y2 side with it being brought into contact with an edge of the band insertion hole 312 on the Y1 side on which the width is provided to be wide, and adjust tightening by the restoring force of the second band portion 40 (a belt portion 44). Further, since the connection portion 310 is provided at the thick portion 345 in which the thickness of the belt portion 34 is increased, even if deformation occurs by a force pulling the second band portion 40, restoration can occur, and thus the deformation can be suppressed.

The projecting bar 314 is pivotally supported on the belt portion 34 by a "spring rod" (not shown) which is inserted into the locking hole 312h provided at one side on the Y2 side at an inner edge of the band insertion hole 312, and a hole (not shown) provided in the projecting bar 314. A portion of the projecting bar 314 is fitted into the concave portion 312c provided at one side on the Y1 side at the inner edge of the band insertion hole 312, whereby the projecting bar 314 is locked to the belt portion 34.

In the projecting bar 314, two bar portions 3141 and 3142 are provided to extend in the Y1 direction from the side of one side on the Y2 side at the inner edge of the band insertion hole 312. Further, in the projecting bar 314, a connection bar 3143 is provided in the direction of the width 30W (the X direction shown in Fig. 4A) of the belt portion 34 intersecting the Y1 direction in which the bar portions 3141 and 3142 extend. The cross-sectional shape of each of the bar portions 3141 and 3142 configuring the projecting bar 314 has a flat shape. The projecting bar 314 has a flat shape, whereby it is possible to increase a contact area when having been inserted into the hole portion 430, suppress the hole portion 430 from being extended, and suppress a shift of the measurement device 1 mounted on a test object by the band section 3 according to this embodiment.

A material configuring the projecting bar 314 is not particularly limited, and it is acceptable if it is a material capable of withstanding the restoring forces of the belt portions 34 and 44. In this embodiment, as for the projecting bar 314, as an example thereof, stainless steel is used. The projecting bar 314 can have toughness to withstand the restoring forces of the belt portions 34 and 44 and corrosion resistance by using stainless steel. Further, the projecting bar 314 is subjected to hairline machining along a direction (the Y direction) in which the bar portions 3141 and 3142 extend. By performing the hairline machining, it is possible to increase the visibility of the projecting bar 314, thereby aiding insertion into the hole portion 430.

In the band section 3 according to this embodiment, the second band portion 40 is inserted into the band insertion hole 312 and the projecting bar 314 is inserted into the hole portion 430 provided in the second band portion 40, whereby the first band portion 30 and the second band portion 40 are connected.

Here, it is required that the band section 3 brings the device main body 2 into close contact with a test object in order to measure the biological information of the test object and the mounting position thereof is not shifted.

For this reason, the hole portions 430 are provided in parallel in the second band portion 40, and the projecting bar 314 is inserted into the hole portion 430, whereby a shift of a mounting position is suppressed (refer to Figs. 6A to 6C) . In the connection portion 310, the bar portions 3141 and 3142 configuring the projecting bar 314 are provided in parallel corresponding to the hole portions 430 and an interval thereof is maintained by the connection bar 3143. In this way, the projecting bar 314 has an H-shape, and thus it is possible to easily insert the projecting bar 314 into the hole portions 430 provided in parallel, while maintaining an interval between the bar portions 3141 and 3142 by the connection bar 3143.

### Cover Portion 320

The cover portion 320 as the first connection portion is provided on one end side (in the Y2 direction shown in Fig. 4A) of the belt portion 34. The locking hole 320h is provided in the cover portion 320.

The cover portion 320 is provided in order to connect the first band portion 30 to the device main body 2 through the mounting member 32. A width 320W of the cover portion 320 and a width 2W of the device main body 2 have approximately the same width (refer to Fig. 1).

More specifically, in a direction (the X direction shown in Figs. 1 to 4B) intersecting a direction (the Y1 direction shown in Figs. 1 to 4A) in which the first band portion 30 extends from the device main body 2, the width 320W of the cover portion 320 and the width 2W of the device main body 2 have approximately the same width. In this way, the device main body 2 and the first band portion 30 can be visible as being visually integrally formed, and thus it is possible to alleviate a feeling of pressure due to mounting. Further, since the device main body 2 does not protrude from the first band portion 30 in the width direction (the X direction), it is possible to suppress the clothes of a test object from being caught in the device main body 2 at the time of mounting.

### Configuration of Second Band Portion

The second band portion 40 shown in Figs. 3, and 6A has the belt portion 44, a cover portion 420 as a second connection portion (an end portion) provided on one end side (the device main body 2 side, in the Y1 direction shown in Figs. 3 and 6A) of the belt portion 44, and the hook portion 410 on the other end side (the opposite side to the device main body 2, in the Y2 direction shown in Figs. 3 and 6A). In addition, in Fig. 6A, a concave portion 400c in which the hook portion 410 is provided and a hole portion 440 are shown and illustration of the hook portion 410 is omitted.

The belt portion 44 has front and back surfaces, and in the following description, the surface coming into contact with a wrist when having been mounted on a test object is described as being referred to as a back surface 44b of the belt portion 44 and the surface being the opposite surface thereof and being visible when having been mounted is described as being referred to as a front surface 44a of the belt portion 44.

### Mounting on Device Main Body 2

The second band portion 40 is mounted on the device main body 2 such that the cover portion 420 covers the lug 204, with the mounting member 42 sandwiched between the lug 204 and the cover portion 320.

The cover portion 420 and the lug 204 are pivotally supported by inserting a "spring rod" (not shown) into a hole 42h provided in the mounting member 42 and a "rod hole 204h" provided in the lug 204 and locking both ends of the spring rod to locking holes 420h provided in the cover portion 420.

### Belt Portion 44

The belt portion 44 shown in Figs. 6A and 6B has stretchability in order to mount the device main body 2 in close contact with the wrist or the like of a test object. The belt portion 44 is made using a material which includes polyurethane resin or silicone resin, thereby having stretchability and flexibility according to the characteristics of the material.

The belt portion 44 is provided with the thickness (in the Z direction shown in Fig. 6B) of a central portion in a direction of the width 40W increased in a cross-section (a cross-section in line F - F' shown in Fig. 6A) in a direction (the second direction, the X direction shown in Fig. 6A) intersecting a direction (the first direction, the Y2 direction shown in Fig. 6A) in which the second band portion 40 extends from the device main body 2. The belt portion 44 is provided with the thickness of the central portion in the direction of the width 40W increased, whereby strength at the time of expansion and contraction and strength at the time of flexion are secured.

The hole portion 430 in which the projecting bar 314 provided at the first band portion 30 is inserted is provided in the belt portion 44. The hole portions 430 are provided in parallel in the direction of the width 40W (the second direction, the X direction shown in Figs. 6A to 6C) and to be aligned to form rows in a direction (the first direction, the Y direction shown in Fig. 6A) in which the belt portion 44 extends. A line spacing of the hole portions 430 may be disposed so as to be almost constant and may also be disposed so that the line spacing becomes shorter as it goes in a direction from the other end to the device main body 2, that is, the Y1 direction. In other words, the line spacing is disposed such that a distance (a second pitch) between the last row in the Y1 direction and a row adjacent thereto becomes narrow compared to a distance (a first pitch) between the last row of the hole portion 430 on the Y2 direction side and a row adjacent thereto. Or, the line spacing is disposed such that the first pitch is wider than the second pitch. Due to such a configuration, even with respect to a test object in which a site of mounting the measurement device 1 is thin, it is possible to finely adjust tightening, and therefore, it becomes easy to set the measurement device 1 with an appropriate strength.

In the hole portion 430, as shown in Figs. 6B and 7A, a hole 431 provided on the front surface 44a side of the belt portion 44 and a hole 432 provided on the back surface 44b side of the belt portion 44 are coaxially provided.

The hole portion 430 has an elliptical shape having a long axis (a major diameter) in the direction of the width 40W (the X direction) of the belt portion 44 and a short axis (a minor diameter) in an orthogonal direction (the Y direction) in which the second band portion 40 extends from the device main body 2, as shown in Fig. 7B. The long axis and the short axis orthogonal to the long axis of the hole portion 430 are provided to be longer (larger) in the hole 432 than in the hole 431.

In addition, the elliptical shape that the holes 431 and 432 have is a shape according to the cross-sectional shape of the projecting bar 314 provided at the connection portion 310 described above and may be appropriately changed in accordance with the cross-sectional shape of the projecting bar 314.

In the band section 3 according to this embodiment, the hole portion 430 is provided such that the size of the hole 431 is approximately 4.0 mm in the long axis direction and approximately 2.5 mm in the short axis direction and the size of the hole 432 is approximately 5.0 mm in the long axis direction and approximately 3.5 mm in the short axis direction. The holes 431 and 432 have an elliptical shape, thereby being able to be easily deformed, and thus the insertion of the projecting bar 314 can be easily performed. In addition, a restoring force after deformation is also excellent.

In the belt portion 44, symbol m is denoted between the hole portions 430 provided in parallel. The symbols m are denoted to form order toward the direction (the Y2 direction shown in Fig. 6A) in which the second band portion 40 extends from the device main body 2. The symbols m are denoted as "1, 2, 3, ..., and 17" in order from the device main body 2 side (the Y1 side shown in Fig. 6A) in a case of being, for example, a numeral. Further, in a case of being an alphabet letter, the symbols can be denoted as "a, b, c, ..." in order from the device main body 2 side. In a case of using an alphabet letter, compared to a case of using a numeral, it is possible to indicate a permutation of a lot of hole portions 430 with the same letter size. That is, it is possible to indicate a permutation of the hole portions 430 of the number equivalent to the twenty-five letters of the alphabet with one letter while maintaining the size of the letter. The font of the symbol m is not particularly limited as long as it is possible to indicate the order of the hole portions 430.

The symbols m are denoted on the belt portion 44, whereby, when constantly mounting the measurement device 1 on a wrist or the like, it is possible to easily identify the optimum position for inserting the projecting bar 314 in order to connect the first band portion 30 and the second band portion 40, and thus it is possible to mount the measurement device 1 without seeking the position of the hole portion 430 for inserting the projecting bar 314 which is optimum for each mounting. Further, the symbol m is denoted between the hole portions 430 provided in parallel, and therefore, it is possible to denote the symbol to the second band portion 40 without newly providing a space (an area).

Further, as shown in Fig. 6C, the concave portion 400c in which a base portion 412 of the hook portion 410 (described later) is fitted and the hole portion 440 in which the pin 414 extending from the base portion 412 is inserted are provided in the belt portion 44.

The concave portion 400c has a concave shape conforming to an outer peripheral edge of the base portion 412 on the front surface 44a of the belt portion 44. Further, a depth 400d of the concave portion 400c is provided to be approximately the same dimension as a thickness 412t of the base portion 412.

The hole portions 440 are provided in parallel in the direction of the width 40W (the X direction) and coaxially with the pins 414 extending from the base portion 412, at a bottom portion of the concave portion 400c, as shown in Fig. 7C.

In the hole portion 440, a hole 441 provided in the bottom portion of the concave portion 400c on the front surface 44a side of the belt portion 44 and a hole 442 provided in the back surface 44b of the belt portion 44 are coaxially provided.

The hole portion 440 has an elliptical shape having a long axis (a major diameter) in the direction of the width 40W of the belt portion 44 and a short axis (a minor diameter) in an orthogonal direction (the Y direction) in which the second band portion 40 extends from the device main body 2. The long axis and the short axis orthogonal to the long axis of the hole portion 440 are provided to be longer (larger) in the hole 442 than in the hole 441.

In addition, the elliptical shape that the holes 441 and 442 have is a shape according to the shape of the pin 414 provided at the hook portion 410 (described later) and may be appropriately changed in accordance with the shape of the pin 414.

In the band section 3 according to this embodiment, the hole portion 440 is provided such that the size of the hole 441 is approximately 4.0 mm in the long axis direction and approximately 2.5 mm in the short axis direction and the size of the hole 442 is approximately 5.0 mm in the long axis direction and approximately 3.5 mm in the short axis direction.

### Hook Portion 410

The configuration of the hook portion 410 will be described using Figs. 8A and 8B while referring to Figs. 1 to 3.

As shown in Figs. 1 to 3, the hook portion 410 is provided on the other end side (in the Y2 direction shown in Figs. 1 to 3) of the belt portion 44.

As shown in Figs. 8A and 8B, the hook portion 410 is provided with the base portion 412 and the pins 414 extending from the base portion 412. The pin 414 is configured to include a first shaft portion 416 and a second shaft portion 418. The hook portion 410 is a member for locking the second band portion 40 inserted into the connection portion 310 to the first band portion 30 by inserting the pins 414 into the hole portions 330 provided in the first band portion 30.

In the hook portion 410, the plurality of pins 414 are provided in accordance with an interval of the hole portions 330 provided in parallel in the direction of the width 30W (the X direction shown in Figs. 1 to 3) of the belt portion 34.

Since the pin 414 is inserted into the hole portion 440 provided in the second band portion 40 and the hole portion 330 of an elliptical shape provided in the first band portion 30, the cross-sectional shape thereof is an elliptical shape. The pin 414 is formed into an elliptical shape having a long axis (a major diameter) in the direction of the width 30W of the belt portion 34 (the direction of the width 40W of the belt portion 44) and a short axis (a minor diameter) in an orthogonal direction (the Y direction) in which the second band portion 40 extends from the device main body 2.

In the hook portion 410, the first shaft portion 416 configuring the pin 414 is provided so as to protrude from the base portion 412. Further, the second shaft portion 418 is provided at one end (in a Z2 direction shown in Figs. 8A and 8B, one end on the opposite side to the base portion 412 from which the first shaft portion 416 extends) of the first shaft portion 416.

A first head portion 417 is provided at the first shaft portion 416. The first head portion 417 is provided on the opposite side (in the Z2 direction shown in Figs. 8A and 8B) to the base portion 412 from which the first shaft portion 416 extends. The first head portion 417 has a curved surface 417r at an outer peripheral edge on the side (in the Z2 direction shown in Figs. 8A and 8B) on which the second shaft portion 418 is provided.

The diameter of the first head portion 417 is provided to be larger than the diameter of the first shaft portion 416. Further, the diameter of the first head portion 417 is provided to be larger than the diameter of the hole 441 and smaller than the diameter of the hole 442.

In the band section 3 according to this embodiment, the dimension (the size) of a shaft portion 416s of the first shaft portion 416 is provided to be approximately 4.0 mm in the long axis direction and approximately 2.5 mm in the short axis direction. Further, the dimension (the size) of the first head portion 417 of the first shaft portion 416 is provided to be approximately 5.5 mm in the long axis direction and approximately 4.0 mm in the short axis direction and to have the radius of curvature R of the curved surface 417r of 0.3 mm.

Further, a second head portion 419 is provided at the second shaft portion 418. The second head portion 419 is provided on the opposite side (in the Z2 direction shown in Figs. 8A and 8B) to the first shaft portion 416 from which the second shaft portion 418 extends. The second head portion 419 has a curved surface 419r at an outer peripheral edge on the opposite side (in the Z2 direction shown in Figs. 8A and 8B) to the side on which the first shaft portion 416 is provided. The diameter of the second head portion 419 is provided to be larger than the diameter of the second shaft portion 418. Further, the diameter of the first head portion 417 is provided to be larger than the diameter of the hole 331 provided in the hole portion 330 in which a tip portion 318 is inserted and smaller than the diameter of the hole 332.

In the band section 3 according to this embodiment, the size of a shaft portion 418s of the second shaft portion 418 is provided to be approximately 3.8 mm in the long axis direction and approximately 2.0 mm in the short axis direction. Further, the size of the second head portion 419 of the second shaft portion 418 is provided to be approximately 4.8 mm in the long axis direction and approximately 3.2 mm in the short axis direction and to have the radius of curvature R of the curved surface 419r of 0.8 mm.

Here, the thickness (in a direction in which the pin 414 extends, the thickness in the Z direction shown in Figs. 8A and 8B) of the base portion 412 and the length (in the direction in which the pin 414 extends, the length in the Z direction shown in Figs. 8A and 8B) of the pin 414 are determined depending on the depths of the hole portion 440 and the hole 331 (the hole portion 330).

The thickness 412t of the base portion 412 in this embodiment is approximately 1.5 mm and is approximately the same as the depth of the concave portion 400c. A length 416L of a shaft portion of the first shaft portion 416 is approximately 1.4 mm and is approximately the same as the depth (the length) of the hole 441. A length (a thickness) 417L of the first head portion 417 of the first shaft portion 416 is approximately 0. 5 mm and is approximately the same as the depth (the length) of the hole 442. A length 418L of a shaft portion of the second shaft portion 418 is approximately 1.9 mm and is approximately the same as the depth (the length) of the hole 331. A length (a thickness) 419L of the second head portion 419 of the second shaft portion 418 is approximately 0.8 mm and is approximately the same as the depth (the length) of the hole 332.

In the hook portion 410, the base portion 412 is fitted into the concave portion 400c provided in the front surface 44a of the belt portion 44. In addition, the pins 414 extending from the base portion 412 are inserted into the hole portions 440 provided in parallel in the direction of the width 40W of the belt portion 44, whereby the hook portion 410 is provided at the belt portion 44. The hook portion 410 is provided such that when having been provided at the belt portion 44, the second shaft portions 418 protrude from the back surface 44b of the belt portion 44. The second shaft portions 418 protrude from the belt portion 44, thereby being able to be inserted into the hole portions 330 provided in the first band portion 30 (the belt portion 34).

In this way, in the first shaft portion 416 inserted into the hole 441, the first head portion 417 is caught in the hole 441, and thus it is possible to suppress the hook portion 410 from being detached from the belt portion 44.

More specifically, due to the curved surface 417r provided at the first head portion 417, it is possible to reduce contact resistance occurring when inserting the first shaft portion 416 into the hole 441. In addition, the curved surface 417r is not provided at the first head portion 417 on the base portion 412 side, and therefore, when extracting the first shaft portion 416 from the hole 441, a larger force than the force at the time of insertion is required. Therefore, it is possible to suppress the hook portion 410 from being detached from the belt portion 44.

Further, in the second shaft portion 418 inserted into the hole portion 330, the second head portion 419 is caught in the hole 331 provided in the first band portion 30, and thus it is possible to lock the second band portion 40 to the first band portion 30. Further, it is possible to suppress detachment of the second band portion 40 locked to the first band portion 30.

More specifically, due to the curved surface 419r provided at the second head portion 419, it is possible to reduce contact resistance occurring when inserting the second shaft portion 418 into the hole 331. In addition, the curved surface 419r is not provided at the second head portion 419 on the first shaft portion 416 side, and therefore, when extracting the second shaft portion 418 from the hole 331, a larger force than the force at the time of insertion is required. Therefore, it is possible to suppress detachment of the second band portion 40 locked to the first band portion 30.

### Cover Portion 420

The cover portion 420 as the second connection portion is provided on one end side (in the Y1 direction shown in Fig. 6A) of the belt portion 44. The locking hole 420h is provided in the cover portion 420.

The cover portion 420 is provided in order to connect the second band portion 40 to the device main body 2 through the mounting member 42. A width 420W of the cover portion 420 and the width 2W of the device main body 2 have approximately the same width (refer to Fig. 1).

More specifically, in a direction (the X direction shown in Figs. 1 to 3, or Figs. 6A to 6C) intersecting the direction (the Y1 direction shown in Figs. 1 to 3, or Fig. 6A) in which the second band portion 40 extends from the device main body 2, the width 420W of the cover portion 420 and the width 2W of the device main body 2 have approximately the same width. In this way, the device main body 2 and the second band portion 40 can be visible as being formed visually integrally, and thus it is possible to alleviate a feeling of pressure due to mounting. Further, since the device main body 2 does not protrude from the second band portion 40 in the width direction (the X direction), it is possible to suppress the clothes of a wearer from being caught in the device main body 2 at the time of mounting.

### Ratio between Widths of Device Main Body 2 and Band section 3

In this embodiment, it is preferable that the ratio between the width of the band section 3 (the width 320W of the cover portion 320 and the width 420W of the cover portion 420) and the width 2W of the device main body 2 is in a range of 0.5 to 1.4.

As a result of response evaluation by the inventors, if the ratio is less than 0.5, the band section 3 is too thin with respect to the device main body 2, and therefore, it does not lead to the solution of a problem of the related art in that there is a feeling of pressure at the time of mounting of the device main body 2. On the other hand, if the ratio exceeds 1.4, the band section 3 is too thick with respect to the device main body 2, and therefore, the size of the measurement device 1 which includes the device main body 2 or the band section 3 becomes too large, and thus a problem in that the size becomes a size which is inappropriate for a test object to continue to wear for a long period of time occurs.

Therefore, in the measurement device 1 according to this embodiment, the ratio between the width (320W and 420W) of the band section 3 and the width 2W of the device main body 2 is provided to be approximately 1.0. In this way, it is possible to stably mount the measurement device 1 on a test object and suppress a feeling of pressure which is given to the test object.

According to the embodiment described above, the following effects can be obtained.

In the band section 3 and the measurement device 1 in which the band section 3 is connected to the device main body 2, the widths 320W and 420W of the band section 3 mounted on the device main body 2 have approximately the same width as the width 2W of the device main body 2. Therefore, a feeling of pressure to a wrist or the like due to prolonged mounting is suppressed, whereby it is possible to stably mount the device main body 2, and a feeling of pressure is suppressed also in terms of a visual sensation, whereby it is possible to make the device main body 2 be viewed as small. Therefore, it is possible to realize the band section 3 in which it is possible to suppress the influence on the biological information of a person on which the device main body 2 is mounted, and the measurement device 1 in which it is possible to obtain biological information with high precision.

In addition, in this embodiment, as the measurement device 1, an example having a function of measuring biological information has been described. However, the invention is not limited thereto, and it is possible to carry out the invention as a portable electronic information device which collects and displays a variety of information. By varying the configuration of the module 20 configuring the device main body 2, it is possible to apply the invention as, for example, a navigation device or a communication device.

## Claims

1. A band for mounting a device main body, comprising:
first and second band portions which are connected to a device main body,
wherein a ratio between widths of the first band portion and the second band portion in a second direction intersecting a first direction in which the first band portion and the second band portion extend from the device main body and a width of the device main body in the second direction is greater than or equal to 0.5 and less than or equal to 1.4.

2. The band according to Claim 1, further comprising:
a connection portion which is provided at an end portion of the first band portion on the opposite side to the device main body and connects the first band portion and the second band portion; and
a hook portion which is provided at an end portion of the second band portion on the opposite side to the device main body and locks the second band portion to the first band portion.

3. The band according to Claim 1 or Claim 2, wherein belt portions having stretchability are provided between an end portion of the first band portion, which is connected to the device main body, and the connection portion and between an end portion of the second band portion, which is connected to the device main body, and the hook portion.

4. The band according to Claim 3, wherein in the belt portions, hole portions in which the connection portion or the hook portion is inserted are provided in parallel in the second direction and to form rows in the first direction.

5. The band according to Claim 4, wherein each of the hole portions has first and second holes provided coaxially,
each of the first hole and the second hole has an elliptical shape having a short axis in the first direction and a long axis in the second direction, and
lengths of the short axis and the long axis of the second hole are larger than lengths of the short axis and the long axis of the first hole.

6. The band according to Claim 4 or Claim 5, wherein in the rows of the hole portions of the first band portion, a distance between a row closest to the device main body side and a row adjacent thereto is narrower than a distance between a row closest to the connection portion side and a row adjacent thereto.

7. The band according to any of Claims 4 to 6, wherein in the rows of the hole portions of the second band portion, a distance between a row closest to the device main body side and a row adjacent thereto is narrower than a distance between a row closest to the hook portion side and a row adjacent thereto.

8. The band according to any of Claims 1 to 7, wherein the first band portion has a thick portion,
the connection portion is provided at the thick portion and
includes a band insertion hole in which the second band portion is inserted, and
a projecting bar which is pivotally supported on one side of the band insertion hole, which extends in the second direction, and is inserted into the hole portion provided in the second band portion, and
the band insertion hole is provided such that a side extending in the second direction, which faces the one side on which the projecting bar is pivotally supported, is long.

9. The band according to any of Claims 2 to 8, wherein the hook portion is provided with a base portion and a pin portion extending from the base portion, and
the pin portion has a first shaft portion extending from the base portion, and a second shaft portion extending from the first shaft portion.

10. The band according to Claim 9, wherein in the pin portion,
a first head portion having a larger diameter than the first shaft portion is provided at the first shaft portion, and
a second head portion having a larger diameter than the second shaft portion is provided at the second shaft portion.

11. The band according to Claim 9 or Claim 10, wherein a length of the second shaft portion in a third direction in which the pin portion extends from the base portion is approximately the same as a depth of the first hole, and a length of the second head portion in the third direction is approximately the same as a depth of the second hole.

12. The band according to Claim 10 or Claim 11, wherein the second shaft portion and the second head portion have an elliptical shape having a short axis in the first direction and a long axis in the second direction, and in a case where the second head portion and the first hole are viewed in plan from the third direction, an area of the second head portion is larger than an area of the first hole.

13. The band according to any of Claims 9 to 12, wherein the second shaft portion and the second head portion have an elliptical shape having a short axis in the first direction and a long axis in the second direction, and the long axis and the short axis of the second head portion are larger than the long axis and the short axis of the first hole.

14. The band according to any of Claims 10 to 13, wherein each of the first head portion and the second head portion has a curved surface in the opposite direction to a direction in which the base portion is provided.

15. The band according to any of Claims 10 to 14, wherein in the belt portions, a thickness in the third direction is approximately the same as a value obtained by adding a length of the second shaft portion and a length of the second head portion.

16. The band according to any of Claims 1 to 15, wherein in the first band portion and the second band portion, the belt portions are made of a material which has stretchability and includes polyurethane resin or silicone resin.

17. A biological information measurement device comprising:
the band according to any of Claims 1 to 16; and
a device main body on which the band is mounted.
